# EUROPEAN PATENT SPECIFICATION

(11) **EP 4 157 944 B1**
(45) Date of publication and mention of the grant of the patent: **01.10.2025**
(21) Application number: 21727483.6
(22) Date of filing: 26.05.2021
(51) Int. Cl.: C09C 1/02, A01N 59/20, A01N 59/16, A61Q 17/00, A01N 55/02, A61K 8/19, A61K 8/36

(54) **HYDROPHOBIC ANTIMICROBIAL AGENTS**
HYDROPHOBE ANTIMIKROBIELLE MITTEL
AGENTS ANTIMICROBIENS HYDROPHOBES

(30) Priority: 27.05.2020 EP 20176909
(43) Date of publication of application: 05.04.2023
(73) Proprietor: Omya International AG, 4665 Oftringen (CH)
(72) Inventor: FTOUNI, Jamal, 4800 Zofingen (CH); GYSIN, Sarah, 4852 Rothrist (CH)
(74) Representative: Maiwald GmbH
(86) International application number: PCT/EP2021/063951
(87) International publication number: WO 2021/239756

(56) References cited:
- WO-A1-2015/011630
- WO-A1-2016/173939
- JP-A- 2005 048 031
- CHENYANG CAO ET AL: "Fabrication of superhydrophobic copper stearate@Fe3O4 coating on stainless steel meshes by dip-coating for oil/water separation", SURFACE AND COATINGS TECHNOLOGY, vol. 349, 1 September 2018 (2018-09-01), AMSTERDAM, NL, pages 296 - 302, XP055742974, ISSN: 0257-8972, DOI: 10.1016/j.surfcoat.2018.06.001

## Description

The present invention relates to hydrophobic antimicrobial agents, a method for the manufacture thereof, their use as fillers, the products comprising the hydrophobic antimicrobial agents, and the use of copper and/or silver salt of at least one fatty acid for surface coating a mineral material.

Microorganisms such as bacteria, algae, fungi, etc. play an important role in many fields, e.g. in the field of polymers, paints, adhesives, coatings, rubber, sealants, cosmetics, textiles, coatings, ceramics, paper products. Apart from their harmful properties for health, they may also cause discoloration, foul odors, or degradation of the material, in which they are contained, or of additives contained therein.

Therefore, it is common practice to add antimicrobial additives to materials, which might be subject to detrimental microbial attacks, e.g. medical devices, toys, sports equipment, appliances, food processing machinery, kitchen utensils, bathroom products, garbage bins, electronic devices, etc. Widely used antimicrobials in this field are based on copper, silver or zinc compounds, and a number of further agents depending on the respective application, e.g. tributyl tin products, or compounds such as 10',10'-oxybisphenox arsine, 2-n-octyl-4-isothiazolin-3-one (OIT) and dichloro-2-n-octyl-4-isothiazolin-3-one (DCOIT), are widely used, especially in polymers.

There are different approaches for providing antimicrobial agents.

For example, WO 2008/144013 A1 relates to bioactive agrichemical concentrates and compositions having improved bioactivity comprising combinations of acid solutions and conventional bioactive agrichemical actives or formulations for preserving or delaying the onset of spoilage in food. This document, however, is silent on the use of fatty acids or another protective.

WO 2012/161603 A2 relates to a hybrid material composed of calcium carbonate microparticles with a polyelectrolyte additive and silver nanoparticles (nAg) embedded in its structure. It does however not mention the use of fatty acids. Moreover, the silver particles are embedded in the calcium carbonate. The calcium carbonate particles may be coated with a polymer layer, however, the silver particles are not in the polymer coating layer.

WO 2014/152586 A1 relates to an anti-microbial additive material and a method for preparing an anti-microbial polymeric material, but is silent on the use of fatty acids. The calcium carbonate particles that are used as filler may be coated with a polymer layer (and not with fatty acids) comprising silver glass and thus the silver particles are in the polymer coating layer.

US 2016/150,793 A1 relates to an additive with biocidal properties based on an active agent with antimicrobial and antifouling properties, wherein the additive corresponds to a supporting material, inert substrate or carrier, which has been modified with antimicrobial agents. This document, however, does not mention the use of fatty acids or another protective.

CN 103788411 A discloses calcium carbonate fillers and particularly relates to a nadic anhydride surface modified calcium carbonate filler, but it is silent on the use of stearic acid or another protective.

US 2010/047,546 A1 relates to a process for preparation of metal-coated non-metallic nano/micro particles. The nano/micro particles are composed of a core and metallic coat over the core using silver or other transition/noble metals. The core of the non-metallic nano/micro particles may be of e.g. calcium carbonate among other minerals. However, the use of fatty acids or another protective coating is not mentioned.

US 2003/091,653 A1 discloses powders with contact biocidal properties comprising a polysaccharide carrying atomic/metallic silver. The preferred polysaccharide is chitin and may be obtained from deproteinated crustacean shells without demineralization, thus being admixed with calcium carbonate. The document is silent on the use of stearic acid or another protective coating.

US 2006/163,136 A1 describes granular plastic particles (pellets) for a filter (apparatus). The plastic pellets are made from any of the following plastics: polyolefins, various kinds of polyacrylates, polyvinyl polymers, nylon, polycarbonates etc., and contain antimicrobial agents that protect the granular media from microbiological growth, for example silver. Furthermore, the plastic pellets may contain fillers such as calcium carbonate. However, neither a specific treatment of the filler, nor fatty acids or any other coating of the filler are mentioned.

JP2005048031A discloses an antibacterial resin, wherein a fatty acid silver salt is mixed with a resin raw material and molded. A robust superhydrophobic surface coated with copper stearate (CS) and F6304 nanoparticles, which was prepared on stainless steel mesh (SSM) by dip-coating method for oil/water separation, is described in the article of Cao and Cheng published in Surface & Coatings Technology 2018, 349, 296-302.

Apart from simply blending the antimicrobial agent into or coating it onto the material to be protected, it has also been tried to combine additives commonly present in the material with antimicrobial agents.

For example, fillers may be upgraded or blended with either an organic or inorganic antimicrobial component. However, if the filler is hydrophobized as it is commonly practiced, or otherwise treated for several reasons, e.g. to ensure a good dispersion, a hydrophobization or other treatment step of the mixture of the blend of fillers and antimicrobial agents is required before incorporating into the material formulation.

For example, WO 2016/173939 A1 relates to a modified mineral-based filler comprising at least one alkaline earth metal carbonate-comprising material, and at least one water insoluble copper salt comprising malachite, which covers at least partially the surface of the at least one alkaline earth metal carbonate-comprising material, and a method of producing the same. According to one embodiment, the modified mineral-based filler is treated with a fatty acid, e.g. stearic acid.

This approach, however, has the drawback that during the treatment, e.g. hydrophobization step, the antimicrobial components are covered by the hydrophobic coating, i.e. may be encapsulated in the hydrophobic coating, and thus isolated, which prevents an efficient contact between the antimicrobial components and the microorganisms.

Thus, apart from the fact that there is generally a continuous need for further antimicrobial agents in a number of applications, it is a first object of the present ivention to provide a readily available antimicrobial agent, which may be used in many applications, such as in the polymer production, which is easily prepared, effective, and even multifunctional.

It is furthermore desirable that the antimicrobial agent provides an effective hydrophobicity and reliable processability.

This object has been solved by a hydrophobic antimicrobial agent, which comprises a mineral material, which is surface coated with a copper and/or silver salt of at least one fatty acid.

Thus, a multifunctional agent is obtained having at least three functions. First, the mineral material may serve as a filler in the material to be protected. Secondly, the filler is hydrophobized by the fatty acid in order to ensure an even distribution in the material to be protected. Thirdly, the filler is provided with an antimicrobial function due to the silver and/or copper ions being localized on the surface of the upgraded filler providing a better contact, i.e. a better antimicrobial performance.

Accordingly, the foregoing and other objects are solved by the subject-matter as defined in the independent claims. Advantageous embodiments of the present invention are defined in the corresponding subclaims.

It should be understood that, for the purpose of the present invention, the following terms have the following meaning:
Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

Accordingly, in a first aspect, the present invention relates to a hydrophobic antimicrobial agent comprising a mineral material, which is surface coated with a copper and/or silver salt of at least one fatty acid as defined in claim 1.

In a preferred embodiment, the natural ground calcium carbonate (GCC) is selected from the group comprising marble, chalk, limestone, and mixtures thereof; and precipitated calcium carbonate (PCC), is selected from the group comprising precipitated calcium carbonates having aragonitic, vateritic or calcitic crystal forms, and mixtures thereof.

"Natural ground calcium carbonate" (GCC) in the meaning of the present invention is a calcium carbonate obtained from natural sources, such as limestone, marble, or chalk, and processed through a wet and/or dry treatment such as grinding, screening and/or fractionating, for example, by a cyclone or classifier.

"Natural ground calcium carbonate" (GCC) preferably is selected from calcium carbonate containing minerals selected from the group comprising marble, chalk, limestone and mixtures thereof. Natural calcium carbonate may comprise further naturally occurring components such as magnesium carbonate, alumino silicate etc.

In general, the grinding of natural ground calcium carbonate may be a dry or wet grinding step and may be carried out with any conventional grinding device, for example, under conditions such that comminution predominantly results from impacts with a secondary body, i.e. in one or more of a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. The wet processed ground calcium carbonate containing mineral material thus obtained may be washed and dewatered by well-known processes, e.g. by flocculation, filtration or forced evaporation prior to drying. The subsequent step of drying (if necessary) may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and calcium hydroxide in an aqueous, semi-dry or humid environment or by precipitation of calcium and carbonate ion source, for example CaCl₂ and Na₂CO₃, out of solution in water. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the precipitated calcium carbonate is precipitated calcium carbonate preferably comprising aragonitic, vateritic or calcitic mineralogical crystal forms or mixtures thereof. PCCs are described, for example, in EP 2 447 213 A1, EP 2 524 898 A1, EP 2 371 766 A1, EP 1 712 597 A1, EP 1 712 523 A1, or WO 2013/142473 A1.

It is especially preferred that the mineral material, especially calcium carbonate, has a weight median particle size *d*₅₀ in the range from 0.1 µm to 20 µm, preferably from 0.2 µm to 10 µm, more preferably from 0.7 µm to 5 µm and most preferably from 1.5 µm to 3.5 µm.

In a further preferred embodiment, the mineral material, especially calcium carbonate, has a top cut particle size (*d*₉₈) of not more than 100 µm, preferably not more than 40 µm, even more preferably not more than 25 µm, most preferably not more than 15 µm, and especially of from 2.9 to 13.8 µm, e.g. from 5.8 to 7.4 µm.

The "particle size" of particulate materials is herein described by its weight-based distribution of particle sizes *dₓ.* Therein, the value *dₓ* represents the diameter relative to which *x* % by weight of the particles have diameters less than *dₓ*. This means that, for example, the *d*₂₀ value is the particle size at which 20 wt% of all particles are smaller than that particle size. The *d*₅₀ value is thus the weight median particle size, i.e. 50 wt% of all particles are smaller than this particle size. For the purpose of the present invention, the particle size is specified as weight median particle size *d*₅₀(wt) unless indicated otherwise.

Furthermore, the mineral material, especially calcium carbonate, may have a specific surface area (BET) of from 0.5 to 50 m²/g, preferably from 1 to 40 m²/g, more preferably from 2 to 30 m²/g, even more preferably from 3 to 20 m²/g, especially from 4 to 15 m²/g and most preferably from 5 to 10 m²/g as measured by the BET nitrogen method.

The mineral material is surface coated with a copper and/or silver salt of at least one fatty acid. Accordingly, the mineral material comprises a surface-treatment layer of a copper and/or silver salt of at least one fatty acid on at least a part of the surface of the mineral material.

The term "a copper and/or silver salt of at least one fatty acid" in the meaning of the present invention means that copper salts of one or more fatty acids, silver salts of one or more fatty acids, and also mixtures of copper salts of at least one fatty acid and silver salts of at least one fatty acid, which may be the same or different, may be used.

In one embodiment of the present invention, the at least one fatty acid comprises, preferably consists of, one fatty acid. Alternatively, the at least one fatty acid comprises, preferably consists of, two or more fatty acids. For example, the at least one fatty acid comprises, preferably consists of, two or three fatty acids.

The at least one fatty acid may be selected from the group comprising aliphatic carboxylic acids having from 5 to 24 carbon atoms, preferably from the group comprising pentanoic acid, hexanoic acid, heptanoic acid, octanoic acid, nonanoic acid, decanoic acid, undecanoic acid, lauric acid, tridecanoic acid, myristic acid, pentadecanoic acid, palmitic acid, heptadecanoic acid, stearic acid, nonadecanoic acid, arachidic acid, heneicosylic acid, behenic acid, tricosylic acid, lignoceric acid and mixtures thereof, more preferably from the group comprising octanoic acid, decanoic acid, lauric acid, myristic acid, palmitic acid, stearic acid, arachidic acid and mixtures thereof and most preferably comprises stearic acid.

The hydrophobic antimicrobial agent according to the invention contains 0.1 to 10 wt%, preferably 0.5 to 8 wt%, more preferably 1 to 6 wt%, even more preferably 2 to 5 wt%, most preferably 3 to 4 wt% of copper and/or silver salt of at least one fatty acid, based on the total weight of the mineral material.

It has turned out that, if less than 0.1 wt% copper and/or silver salt of at least one fatty acid are used, the moisture pickup susceptibility becomes inacceptably high. On the other hand, if more than 10 wt% copper and/or silver salt of at least one fatty acid are used, the moisture pickup susceptibility is not significantly decreased, but undesirable agglomerates are formed, which have a negative impact on the coating of the mineral material.

Accordingly, a proper selection of the amount of stearate appears to be crucial for obtaining a hydrophobic antimicrobial agent having an effective antimicrobial activity, a satisfying hydrophobicity, and being still processable.

In an especially preferred embodiment, the hydrophobic antimicrobial agent has a moisture pickup susceptibility of from 0.05 to 1 mg of H₂O/g of solid, preferably from 0.075 to 0.75 mg of H₂O/g of solid, more preferably from 0.1 to 0.5 mg of H₂O/g of solid, even more preferably from 0.15 to 0.4 mg of H₂O/g of solid, and most preferably from 0.2 to 0.3 mg of H₂O/g of solid.

The "moisture pickup susceptibility" of a material refers to the amount of moisture absorbed on the surface of said material within a certain time upon exposure to a defined humid atmosphere and is expressed in mg/g. The "normalized moisture pickup susceptibility" of a material refers to the amount of moisture absorbed on the surface of said material within a certain time upon exposure to a defined humid atmosphere and is expressed in mg/m².

The moisture pick up susceptibility of a material as referred to herein is determined in mg moisture/g after exposure to an atmosphere of 10 % and 85 % relative humidity, respectively, for 2 hours at a temperature of +23°C (± 2°C). For this purpose, the sample is first kept at an atmosphere of 10 % relative humidity for 2 hours, then the atmosphere is changed to 85 % relative humidity at which the sample is kept for another 2 hours. Finally, the atmosphere is changed to 10 % of humidity for 30 minutes. The weight increase between 10 % and 85 % relative humidity is then used to calculate the moisture pick-up in mg moisture/g of sample.

The moisture pick up susceptibility in mg/g divided by the specific surface area in m² (BET method) corresponds to the "normalized moisture pick up susceptibility" expressed in mg/m² of sample.

A further aspect of the present invention is a method for the manufacture of the hydrophobic antimicrobial agent as described above, which is characterized by the steps of
a) providing at least one fatty acid,
b) providing at least one copper and/or silver salt being soluble in a non-aqueous solvent,
c) providing a mineral material,
d) dissolving the at least one fatty acid of step a) in a non-aqueous solvent,
e) dissolving the at least one copper and/or silver salt of step b) in a non-aqueous solvent,
f) combining the solutions of step d) and e),
g) precipitating the copper and/or silver salt of the at least one fatty acid by adding water to the combined solutions of step f),
h) separating the precipitated copper and/or silver salt of at least one fatty acid from the reaction mixture of step g)
i) surface coating the mineral material of step c) with the precipitated copper and/or silver salt of at least one fatty acid of step h), wherein
   the mineral material is selected from the group comprising natural ground calcium carbonate (GCC) and precipitated calcium carbonate (PCC),
   the copper and/or silver salt being soluble in a non-aqueous solvent of step b) is selected from the group comprising copper acetate, copper pyrophosphate, copper nitrate, copper chloride, copper oxalate, silver chloride, silver acetate, silver nitrate, and silver sulfate, their hydrates, and mixtures thereof,
   the non-aqueous solvent is selected from the group comprising ethanol, methanol, acetone, acetonitrile, dioxane, ethyl acetate, toluene, xylene, tetrahydrofurane, and mixtures thereof,
   the combination step f) is carried out under stirring, and
   in coating step i), the mineral material of step c) is combined with the copper and/or silver salt of at least one fatty acid of step h) under stirring, wherein the copper and/or silver salt of at least one fatty acid of step h) is combined with the mineral material of step c) in an amount of from 0.1 to 10 wt%, based on the total weight of the mineral material.

It is preferred that the at least one fatty acid of step a) is selected from the group comprising aliphatic carboxylic acids having from 5 to 24 carbon atoms, preferably from the group comprising pentanoic acid, hexanoic acid, heptanoic acid, octanoic acid, nonanoic acid, decanoic acid, undecanoic acid, lauric acid, tridecanoic acid, myristic acid, pentadecanoic acid, palmitic acid, heptadecanoic acid, stearic acid, nonadecanoic acid, arachidic acid, heneicosylic acid, behenic acid, tricosylic acid, lignoceric acid and mixtures thereof, more preferably from the group comprising octanoic acid, decanoic acid, lauric acid, myristic acid, palmitic acid, stearic acid, arachidic acid and mixtures thereof and most preferably comprises stearic acid.

The at least one copper and/or silver salt being soluble in a non-aqueous solvent of step b) is used for the manufacture of the copper and/or silver salt of the at least one fatty acid, and preferably is not selected from copper and/or silver salts of fatty acids.

Rather, the copper and/or silver salt being soluble in a non-aqueous solvent of step b) is selected from the group comprising copper acetate, copper pyrophosphate, copper nitrate, copper chloride, copper oxalate, silver chloride, silver acetate, silver nitrate, and silver sulfate, their hydrates, and mixtures thereof.

In an especially preferred embodiment, the at least one copper and/or silver salt of step b) is provided in an amount of from 0.5 to 75 wt%, preferably in an amount of from 1 to 70 wt%, more preferably in an amount of from 5 to 65 wt%, even more preferably in an amount of from 10 to 55 wt%, most preferably in an amount of from 15 to 50 wt%, especially preferably in an amount of from 20 to 40 wt%, e.g. 30 wt%, based on the total weight of the at least one fatty acid of step a).

The non aqueous solvent(s) mentioned in step b), d) and e) is/are selected from the group comprising ethanol, methanol, acetone, acetonitrile, dioxane, ethyl acetate, toluene, xylene, tetrahydrofurane, and mixtures thereof, and especially preferably is ethanol. The non aqueous solvent of step d) and e) may be the same or different solvents, provided that the at least one fatty acid, of step a) and the at least one copper and/or silver salt of step b) both are soluble in said solvents and/or a mixture of said solvents.

A "solution" as referred to herein is understood to be a single phase mixture of one or more specific solvent(s) and one or more specific solute(s), for example a single phase mixture of an active ingredient and water. The term "dissolved" as used herein thus refers to the physical state of a solute in a solution.

Dissolution steps d) and/or e), independently from each other, may be carried out at a temperature of from 25 to 80 °C, preferably of from 30 to 70 °C, more preferably of from 40 to 60 °C, most preferably at 50 °C.

Furthermore, dissolution steps d) and/or e), independently from each other, may be carried out under stirring for a certain time, e.g. for a few seconds up to several hours, e.g. for 5 seconds to 180 min, preferably 1 min to 120 min, more preferably 5 min to 100 min, even more preferably 10 min to 80 min, especially 20 min to 60 min, most preferably 30 min to 40 min, in any event until a solution is formed. Otherwise, the amount of non aqueous solvent may be increased.

In dissolution steps d) and/or e), the non aqueous solvent is present in an amount sufficient to dissolve the at least one fatty acid of step a) and the at least one copper and/or silver salt of step b), respectively.

For example, if stearic acid is used as at least one fatty acid, and ethanol is used as the non aqueous solvent in step d), stearic acid may be dissolved in a concentration of 333 g/l.

Furthermore, if the at least one copper salt, e.g., is copper acetate monohydrate, and ethanol is used as the non aqueous solvent in step e), copper acetate monohydrate may be dissolved in a concentration of e.g. from 10 g/l to 200 g/l, preferably 20 g/l to 160 g/l, more preferably 40 g/l to 80 g/l, e.g. 60 g/l, optionally at elevated temperatures as mentioned above.

In another preferred embodiment, if the at least one silver salt is silver nitrate and ethanol is used as the non aqueous solvent in step e), silver nitrate may be dissolved in a concentration of e.g. from 1 g/l to 30 g/l, preferably 4 g/l to 15 g/l, more preferably 8 g/l to 10 g/l, optionally at elevated temperatures as mentioned above.

Subsequently, the solutions of steps d) and e) are combined in step f), e.g. by adding the solution of step d) to the solution of step e), or by adding the solution of step e) to the solution of step d).

Combination step f) preferably is carried out under stirring at a temperature of from 25 to 80 °C, more preferably of from 30 to 70 °C, even more preferably of from 40 to 60 °C, most preferably at 50 °C, and especially preferably at the temperature of any one of steps d) or e).

Combining in the meaning of the present invention, e.g. with respect to step f), means any action by which two or more compounds, such as e.g. the solutions of step d) and e) are blended. The combination step may be carried out e.g. by stirring, mixing, kneading, pouring one compound or solution or dispersion or any other formulation thereof into the other compound or solution or dispersion or any other formulation thereof, applying ultrasound, vibration, or in laminar, turbulant or transitional flow regimens, etc.

After combining the solutions of step d) and e), it may be preferred to further stir the mixture, preferably for a few seconds up to several hours, e.g. for 5 seconds to 180 min, preferably 10 min to 160 min, more preferably 20 min to 120 min, even more preferably 25 min to 100 min, especially 30 min to 80 min, most preferably 40 min to 60 min.

Stirring is advantageously carried out at the temperature of the combination step, but may also be carried out at higher or lower temperatures.

If, in combination step f), any one of the compounds should precipitate, additional non aqueous solvent may be added in order redissolve the respective compound. Any one of the compounds preferably are dissolved before precipitation step g) is carried out.

Subsequently, in step g), the copper and/or silver salt of the at least one fatty acid is obtained by precipitating it by the addition of water to the combined solutions of step f).

Also precipitation step g) may be carried out under stirring, preferably under stirring at a temperature of from 25 to 80 °C, more preferably of from 30 to 70 °C, even more preferably of from 40 to 60 °C, most preferably at 50 °C, and especially preferably at the temperature of step f), or a lower temperature.

After precipitating the copper and/or silver salt of the at least one fatty acid from the combined solutions of step f), it may be preferred to let the mixture cool down and/or to further stir the mixture. In this respect, it is especially preferred to stir the mixture for a few seconds up to several hours, e.g. for 5 seconds to 180 min, preferably 10 min to 160 min, more preferably 20 min to 120 min, even more preferably 25 min to 100 min, especially 30 min to 80 min, most preferably 40 min to 60 min.

In precipitation step g), water is added in an amount sufficient to precipitate the copper and/or silver salt of the at least one fatty acid. For example, an amount half as much as the one of the combined non aqueous solvent may be appropriate.

Stirring may be carried out by any equipment suitable therefor, e.g. by a magnetic stirrer or a high speed mixer.

After precipitation step g), and optionally further stirring, the obtained copper and/or silver salt of the at least one fatty acid is separated from the reaction mixture, especially from the liquid phase, and, optionally, washed with water.

Separation of the copper and/or silver salt of the at least one fatty acid may be carried out by any suitable means known in the art, e.g. by filtration, decantation or flotation.

The copper and/or silver salt of the at least one fatty acid may be dried. Drying may be carried out by conventional drying methods, e.g. by thermal drying methods such as in an oven, in a special embodiment under vacuum or static air, by jet or spray drying.

Unless specified otherwise, the term "drying" refers to a process according to which at least a portion of solvent such as water is removed from a material to be dried such that a constant weight of the obtained "dried" material at 150°C is reached.

Drying according to the invention may, however, also be carried out at lower temperatures, depending on the solvents used and/or the drying time, e.g. at a temperature of 50 °C.

A "dried" or "dry" material may be defined by its total moisture content which, unless specified otherwise, is less than or equal to 5.0 wt.%, preferably is less than or equal to 1.0 wt%, more preferably less than or equal to 0.5 wt%, even more preferably less than or equal to 0.2 wt%, and most preferably between 0.05 and 0.1 wt%, based on the total weight of the dried material.

The obtained copper and/or silver salt of the at least one fatty acid may be deagglomerated. This deagglomeration step may be carried out manually, or in any equipment suitable therefor, preferably in a centrifuge, pin mill, high speed mixer or attritor mill.

The obtained copper and/or silver salt of the at least one fatty acid may contain a certain amount of the free fatty acid.

If the copper and/or silver salt of the at least one fatty acid is dried, the deagglomeration step may be carried out before, during and/or after drying.

Finally, in step i), the mineral material of step c) is surface coated with the precipitated copper and/or silver salt of the at least one fatty acid obtained in step h).

Suitable equipment therefor is any equipment commonly known for this purpose, e.g. a Lödige ploughshare^{®} mixer, or a Somakon MP - LB mixer.

Surface coating is achieved by combining the mineral material of step c) with the copper and/or silver salt of at least one fatty acid of step h) under stirring. The coating may be carried out as a wet coating process or dry coating process, wherein dry coating is preferred.

According to step i) of the inventive process, the mineral material of step c) may be contacted by mixing it, in one or more steps, with the the precipitated copper and/or silver salt of at least one fatty acid of step h) or mixtures thereof. The coating step i) may also be referred to as the contacting step or mixing step.

Mixing, and consequently coating, may be carried out by stirring, rolling, kneading, exposure to ultrasound, etc.. Mixing devices (mixers) types include: Propeller, turbo, blade, trough, planetary, friction, screw, roller, centrifugal, counter-current, jet, drum, cone, tumbling, gyro, cooling, vacuum , flow, gravity, fluid and pneumatic mixers. Mixing is one of the oldest unit-operations in the industries and well known to the skilled person.

If the surface coating of the mineral material of step c) with the precipitated copper and/or silver salt of at least one fatty acid of step h) according to step i) takes place under mixing conditions, the skilled person will adapt these mixing conditions (such as the configuration of mixing pallets and mixing speed) according to the available process equipment.

In one preferred embodiment of the present invention, the inventive process may be a continuous process. In this case, it is possible to contact the mineral material of step c) with the precipitated copper and/or silver salt of at least one fatty acid of step h) in a constant flow, so that a constant concentration of the precipitated copper and/or silver salt of at least one fatty acid of step h) is provided during step i).

Alternatively, the mineral material of step c) is contacted with the precipitated copper and/or silver salt of at least one fatty acid of step h) in one step, wherein said precipitated copper and/or silver salt of at least one fatty acid of step h) is preferably added in one portion.

In another embodiment of the present invention, the inventive process may be a batch process, i.e. the mineral material of step c) is contacted with precipitated copper and/or silver salt of at least one fatty acid of step h) in more than one steps, wherein said precipitated copper and/or silver salt of at least one fatty acid of step h) is preferably added in about equal portions. Alternatively, it is also possible to add the precipitated copper and/or silver salt of at least one fatty acid of step h) in unequal portions to mineral material of step c), i.e. in larger and smaller portions.

In coating step i), the copper and/or silver salt of at least one fatty acid of step h) may be combined with the mineral material of step c) in an amount of from 0.25 to 8 wt%, more preferably in an amount of from 0.5 to 6 wt%, even more preferably in an amount of from 0.75 to 4 wt%, most preferably in an amount of from 1 to 2 wt% based on the total weight of the mineral material.

Coating step i) may be carried out at a temperature of from 25 to 180 °C, preferably of from 40 to 160 °C, more preferably of from 60 to 130 °C, most preferably of from 80 to 110 °C.

In another preferred embodiment coating step i) may be carried out at a temperature above the melting point of the copper and/or silver salt of at least one fatty acid of step h).

After combining the mineral material of step c) with the copper and/or silver salt of at least one fatty acid of step h), the mixture may be stirred for a few seconds up to one or two hours, e.g. for 5 seconds to 180 min, preferably 10 min to 160 min, more preferably 20 min to 120 min, even more preferably 25 min to 100 min, especially 30 min to 80 min, most preferably 40 min to 60 min.

The obtained product is at least partially, preferably completely, coated with the copper and/or silver salt of at least one fatty acid, and provides very good hydrophobic as well as antimicrobial properties.

A still further aspect of the invention is the use of the hydrophobic antimicrobial agent as a filler. Especially preferred is the use of the hydrophobic antimicrobial agent as a filler in polymers, paints, adhesives, coatings, rubber, sealants and cosmetics.
Generally, the hydrophobic antimicrobial agent of the invention may be used in any application requiring a hydrophobic filler.

Accordingly, products, preferably polymer, paint, adhesive, coating, rubber, sealant and cosmetic products, comprising a hydrophobic antimicrobial agent according to the present invention, are a still further aspect of the present invention.

Finally, the present invention relates to the use of a copper and/or silver salt of at least one fatty acid as described above, and preferably as obtained by the method of manufacture according to the invention, for surface coating a mineral material.

The following examples and tests will illustrate the present invention, but are not intended to limit the invention in any way.

### EXAMPLES

### 1. Analytical Methods

### Particle size distribution (mass % particles with a diameter < X) and weight median diameter (d₅₀) of a particulate material

Particle sizes were determined by using a Sedigraph^{™} 5120 instrument of Micromeritics Instrument Corporation. The method and the instrument are known to the skilled person and are commonly used to determine the particle size of fillers and pigments. The measurements were carried out in an aqueous solution of 0.1 wt% Na₄P₂O₇. The samples are dispersed using a high speed stirrer and supersonics.

### BET specific surface area of a material

The "specific surface area" (expressed in m²/g) of a material as used throughout the present document is determined by the Brunauer Emmett Teller (BET) method with nitrogen as adsorbing gas and by use of a ASAP 2460 instrument from Micromeritics. The method is well known to the skilled person and defined in ISO 9277:2010. Samples are conditioned at 100 °C under vacuum for a period of 60 min prior to measurement. The total surface area (in m²) of said material can be obtained by multiplication of the specific surface area (in m²/g) and the mass (in g) of the material.

### Moisture pick up susceptibility

The moisture pick up susceptibility of a material as referred to herein is determined in mg moisture/g after exposure to an atmosphere of 10 % and 85 % relative humidity, respectively, for 2 hours at a temperature of +23°C (± 2°C). For this purpose, the sample is first kept at an atmosphere of 10 % relative humidity for 2 hours, then the atmosphere is changed to 85 % relative humidity at which the sample is kept for another 2 hours. Finally, the atmosphere is changed to 10 % of humidity for 30 minutes. The weight increase between 10 % and 85 % relative humidity is then used to calculate the moisture pick-up in mg moisture/g of sample.

### X-ray diffraction (XRD)

XRD experiments are performed on the samples using rotatable PMMA holder rings. Samples are analysed with a Bruker D8 Advance powder diffractometer obeying Bragg's law. This diffractometer comprises a 2.2 kW X-ray tube, a sample holder, a θ-θ-goniometer, and a VÅNTEC-1 detector. Nickel-filtered Cu-Kα radiation is employed in all experiments. The profiles are chart recorded automatically using a scan speed of 0.7° per min in 2 θ (XRD GV_7600). The resulting powder diffraction patterns are classified by mineral content using the DIFFRACsuite software packages EVA and SEARCH, based on reference patterns of the ICDD PDF-2 database (XRD LTM_7603).

Quantitative analysis of diffraction data refers to the determination of amounts of different phases in a multi-phase sample and has been performed using the DIFFRACsuite software package TOPAS (XRD LTM_7604). In detail, quantitative analysis allows to determine structural characteristics and phase proportions with quantifiable numerical precision from the experimental data itself. This involves modelling the full diffraction pattern using the Rietveld approach such that the calculated pattern(s) duplicates the experimental one.

### Inductively Coupled Plasma (ICP) analysis

Prior to the analysis, 0.2 g of the samples (i.e. the copper and/or silver salt of at least one fatty acid) were digested in a mixture containing 5 ml of 69 % HNO₃, 2 ml 30 % H₂O₂ and 3 ml H₂O. The samples were decomposed by microwave assisted digestion at elevated temperature and pressure, which is generated by the vapor pressure of the heated liquids contained in the chamber, using the following microwave temperature program: from 20 °C to 85 °C for 7 min, then from 85 °C to 160 °C for 7 min and, finally, from 160 °C to 180 °C for 12 min.

After the decomposition, the solutions were filled up to 50 ml with distilled water and analyzed by ICP-OES Optima 8300 from Perkin Elmer.

### 2. Material

### Mineral Material

Ground natural calcium carbonate (*d*₅₀ = 1.8 µm, *d*₉₈ = 5.7 µm; BET = 3.4 m²/g; moisture pickup susceptibility = 1.3 mg/g solid)
**Fatty acid**
   Stearic acid, reagent grade 95% (from Sigma Aldrich; CAS No. 57-11-4)
**Copper** / **Silver salt**
   Copper acetate monohydrate (from Sigma Aldrich; CAS No. 6046-93-1)
   Silver nitrate (from Sigma Aldrich; CAS No. 7761-88-8)
**Non aqueous solvent**
   Ethanol (from Sigma Aldrich; CAS No. 64-17-5)
**Other solvent**
   Distilled water
**Polymer**
   Polyethylene LLDPE Dowlex2631.10UE supplied by Resinex

### 3. Preparation

### 3.1. Preparation of copper / silver salt of a fatty acid

### 3.1.1. Copper stearate

Copper stearate was prepared by first dissolving stearic acid in ethanol in a 1 l round bottom flask in the amounts given in table 1 at 50°C until a complete dissolution of the latter was achieved. At the same time, in a different bottom flask, copper acetate monohydrate was dissolved in ethanol in the amounts given in table 1, at the same temperature. Once both components were completely dissolved, the copper acetate monohydrate solution was added to the stearic acid solution dropwise and mixed for 2 hours at 50 °C. Finally, water in the amount given in table 1 was added to the mixture, precipitating the final product. The product was separated by filtration under vacuum using Whatman filters grade 589, washed with water and dried in an oven at 50°C overnight. The product was deagglomerated manually.

**Table 1**

| **Sample** | **Stearic acid (g)** | **Ethanol (ml)** | **Copper acetate monohydrate (g)** | **Ethanol (ml)** | **Water for the precipitation (ml)** |
|---|---|---|---|---|---|
| 1 | 100 | 300 | 63.5 | 400 | 350 |
| 2 | | | 15.88 | 200 | 250 |
| 3 | | | 3.97 | 100 | 200 |
| 4 | | | 0.99 | 50 | 175 |

The obtained copper stearate treatment agents were analyzed using XRD and ICP techniques (cf. table 2).

**Table 2**

| **Sample** | **XRD data (%)** | | | | **ICP analysis (%)** |
|---|---|---|---|---|---|
| | **Stearic acid** | **Copper stearate** | **Amorphous content** | **Σ** | **Amount of Cu** |
| 1 | 14 | 37 | 49 | 100 | 13.6 |
| 2 | 26 | 34 | 40 | 100 | 5.0 |
| 3 | 45 | 17 | 37 | 99 | 1.3 |
| 4 | 54 | 6 | 40 | 100 | 0.3 |
| Stearic acid reagent grade, 95% | 80 | 0 | 15 | 95 | 0 |

### 3.1.2. Silver stearate

Silver stearate was prepared by first dissolving stearic acid in ethanol in a 5 I bottom flask in the amounts given in table 3 at 50°C until a complete dissolution of the latter was achieved. At the same time, in a different bottom flask, silver nitrate was dissolved in ethanol in the amounts given in table 3, at the same temperature. Once both components were completely dissolved, the silver nitrate solution was added to the stearic acid solution and mixed for 2 hours at 50 °C. Finally, water in the amount given in table 3 was added to the mixture, precipitating the final product. The product was separated by filtration under vacuum using Whatman filters grade 589, washed with water and dried in an oven at 50°C overnight. The product was deagglomerated manually.

**Table 3**

| **Sample** | **Stearic acid (g)** | **Ethanol (ml)** | **Silver nitrate (g)** | **Ethanol (ml)** | **Water for the precipitation (ml)** |
|---|---|---|---|---|---|
| 5 | 100 | 300 | 59.5 | 2000 | 1150 |
| 6 | | | 14.88 | 1000 | 650 |
| 7 | | | 3.72 | 500 | 400 |
| 8 | | | 0.93 | 250 | 275 |

The obtained silver stearate treatment agents were analysed using XRD and ICP techniques (cf. table 4).

**Table 4**

| **Sample** | **XRD data (%)** | | | | **ICP analysis (%)** |
|---|---|---|---|---|---|
| | **Stearic acid** | **Silver stearate** | **Amorphous content** | **Σ** | **Amount of Ag** |
| 5 | 15 | 48 | 37 | 100 | 19.3 |
| 6 | 40 | 30 | 30 | 100 | 4.7 |
| 7 | 56 | 16 | 28 | 100 | 1.5 |
| 8 | 68 | 5 | 27 | 100 | 0.3 |
| Stearic acid reagent grade, 95% | 80 | 0 | 15 | 95 | 0 |

### 3.2. Surface coating of calcium carbonate with copper / silver salt of a fatty acid

Each one of samples 1 to 8 as well as, as a comparative sample, stearic acid, were coated onto calcium carbonate, as follows:
Calcium carbonate was dried for 16 hours at 160°C. The coater (a Lödige ploughshare^{®} mixer of 5 litres) as heated to 110°C for 1 hour. Then, 1163 g of the dried calcium carbonate was put inside the coater and stirred for 5 minutes at 100 rpm. Subsequently, the respective sample was added in an amount based on the weight of the calcium carbonate as mentioned in table 5 below, and both components, calcium carbonate and the respective sample, were mixed for further 30 min. at 1000 rpm. The mixer was cooled down to 60 °C and maintained at 100 rpm for 1 hour. Finally, the coated calcium carbonate is removed.

### 4. Characterization

### 4.1. Moisture pickup susceptibility

Subsequently, hydrophobicity of the coated calcium carbonate was evaluated by determination of the respective moisture pickup susceptibility.

**Table 5**

| Sample No. | wt% | Moisture pickup susceptibility (mg of H₂O/g of solid) | Sample No. | wt% | Moisture pickup susceptibility (mg of H₂O/g of solid) |
|---|---|---|---|---|---|
| Stearic acid | 0.5 wt% | 0.75 | Stearic acid | 0.5 wt% | 0.75 |
| Stearic acid | 1 wt% | 0.21 | Stearic acid | 1 wt% | 0.21 |
| Stearic acid | 2 wt% | 0.28 | Stearic acid | 2 wt% | 0.28 |
| 1 | 1 wt% | 0.75 | 5 | 1 wt% | 0.79 |
| 1 | 2 wt% | 0.47 | 5 | 2 wt% | 0.54 |
| 1 | 5 wt% | 0.55 | 5 | 3.5 wt% | 0.36 |
| 2 | 1 wt% | 0.52 | 6 | 1 wt% | 0.44 |
| 2 | 2 wt% | 0.19 | 6 | 2 wt% | 0.23 |
| 3 | 1 wt% | 0.27 | 7 | 1 wt% | 0.28 |
| 3 | 2 wt% | 0.16 | 7 | 2 wt% | 0.19 |
| 4 | 1 wt% | 0.18 | 8 | 1 wt% | 0.18 |
| 4 | 2 wt% | 0.16 | 8 | 2 wt% | 0.15 |
| 4 | 5 wt% | 0.25 | | | |
| 4 | 10 wt% | 0.25 | | | |
| 4 (comparative) | 0.05 wt% | 1.39 | | | |
| 4 (comparative) | 12 wt% | 0.22* | | | |

| | | | | | |
|---|---|---|---|---|---|
| *agglomerates were formed; an amount of copper stearate remained in the coater drum | | | | | |

From table 5, it can be taken that calcium carbonate coated with inventive copper or silver stearates, provides comparable results as regards hydrophobicity at sometimes somewhat higher but acceptable amounts considering that at the same time antimicrobial protection is provided.

As regards the selection of proper amounts, it can be taken from the comparative examples that at an amount of 0.05 wt% copper stearate, the moisture pickup susceptibility significantly increased, whereas at an amount of 12 wt% no significant decrease of the moisture pickup susceptibility could be observed, but instead an agglomeration of the copper stearate, part of which remained in the coater drum.

Accordingly, a proper selection of the amount of stearate appears to be crucial for obtaining a hydrophobic antimicrobial agent having an effective antimicrobial activity, a satisfying hydrophobicity, and being still processable.

### 4.2. Antimicrobial Activity

In the following tests, antimicrobial activity of silver stearate and copper stearate coated onto calcium carbonate and embedded in a polyethylene formulation was evaluated in view of the bacterial test organism Escherichia coli (DSM 1576 / JISZ test 2801).

### 4.2.1. Sample preparation

### Embedment of metal stearate coated calcium carbonate into polyethylene formulation

The following samples were prepared:
- 2 test pieces (4 x 4 cm²) of non-treated calcium carbonate embedded in a polyethylene formulation
- 5 test pieces (4 x 4 cm²) of metal stearate coated calcium carbonate as described above and embedded in a polyethylene formulation

Pellets were produced on a twin-screw extruder 25:1 from Three Tec (Extruder Type ZE12, die: 0.5 mm) with the following line settings:

| | |
|---|---|
| - Extruder temperatures: | 20 °C (feeding) - 190 °C / 210 °C / 210 °C / 190 °C |
| - Feeding speed: | 7.5 - 10 % |
| - Screw speed: | 25 - 35 rpm |
| - Conveyor speed: | 1.5 - 2.25 rpm |
| - Cut speed: | 14 - 22 rpm |

The polymer used is a linear low-density polyethylene (LLDPE) that can be obtained from Resinex under the tradename Dowlex2631.10UE.

### Injection

Plates were made in a mini injection moulder IM12 from Xplore Instruments B.V. The 8 mL (40 x 40 x 5 mm) barrel (square) is filled in with pellets produced as described previously with the following settings:

| | |
|---|---|
| - Barrel temperature: | 220 °C |
| - Preheating time: | 2 min |
| - Mould temperature: | 80 °C |
| - Pressure (Time): | 7 bars (2 s), from 7 to 8 bars (3 s), 8 bars (holding pressure, 12 s) |

### 4.2.2. Results

An antimicrobial surface activity test according to ISO 22196/ JIS Z 2801:2000 (Japanese Industrial Standard procedure) and described in LTM_1041 using the test organism Escherichia coli (DSM 1576) was conducted. The following amendments from the test set-up were done:
1 . Two test pieces per metal stearate were available for the experiment. Results were obtained at 24 and 72 hours of incubation. The series of results obtained at 24 and 72 hours, were performed independently.
2. The size of the test pieces was 4 x 4 cm² (instead of 5 x 5 cm²). However, the area finally being in contact with the test organism was according to the norm (4 x 4 cm²).
3. The test pieces were cleaned with 70% ethanol in water (w/w) before usage and air dried.
4. Test bacteria were not released from the test pieces using a sterile stomacher pouch but instead by adding 10 ml SCDLP broth directly into the petri dish and by gently massaging the test piece with a Drigalski spatula.

**Table 6**

| CaCO₃ filler coated with x wt% of copper stearate sample No. | Incubation time (h) | Antimicrobial reduction (%) a | CaCO₃ filler coated with x wt% of copper stearate sample No. | Incubation time (h) | Antimicrobial reduction (%) b |
|---|---|---|---|---|---|
| Stearic acid (control) | 24 | - | Stearic acid (control) | 72 | - |
| 2 (2 wt%) | | 9.5 | 2 (2 wt%) | | 21.1 |
| 3 (1 wt%) | | 8.9 | 3 (1 wt%) | | 15.8 |
| 3 (2 wt%) | | 14.7 | 3 (2 wt%) | | 9.8 |
| 4 (1 wt%) | | 9.0 | 4 (1 wt%) | | 14.4 |
| 4 (2 wt%) | | 17.2 | 4 (2 wt%) | | 42.3 |

| | | | | | |
|---|---|---|---|---|---|
| ^{a} (Log (cfu/ml) of samples at t = 24h) * 100 / ((Log (cfu/mL) of 'control sample' at t = 24h ^{b} (Log (cfu/ml) of samples at t = 72h) * 100 / ((Log (cfu/mL) of 'control sample' at t = 72h | | | | | |

**Table 7**

| CaCO₃ filler coated with x wt% of silver stearate sample No. | Incubation time (h) | Antimicrobial reduction (%) a | CaCO₃ filler coated with x wt% of silver stearate sample No. | Incubation time (h) | Antimicrobial reduction (%) b |
|---|---|---|---|---|---|
| Stearic acid (control) | 24 | - | Stearic acid (control) | 72 | - |
| 6 (2 wt%) | | 29.7 | 6 (2 wt%) | | 25.0 |
| 7 (1 wt%) | | 13.3 | 7 (1 wt%) | | 8.8 |
| 7 (2 wt%) | | 26.6 | 7 (2 wt%) | | 32.7 |
| 8 (1 wt%) | | 14.5 | 8 (1 wt%) | | 9.6 |
| 8 (2 wt%) | | 8.1 | 8 (2 wt%) | | 3.5 |

| | | | | | |
|---|---|---|---|---|---|
| ^{a} (Log (cfu/ml) of samples at t = 24h) * 100 / ((Log (cfu/mL) of 'control sample' at t = 24h ^{b} (Log (cfu/ml) of samples at t = 72h) * 100 / ((Log (cfu/mL) of 'control sample' at t = 72h | | | | | |

The coating containing metal stearate supported on ground natural calcium carbonate and embedded in a polyethylene formulation showed clear antimicrobial activity against the test organism Escherichia coli. Reduction in bacteria growth was seen in both cases, using silver and copper stearate coated calcium carbonate.

## Claims

1. A hydrophobic antimicrobial agent,
**characterized in that** it comprises a mineral material, which is surface coated with a copper and/or silver salt of at least one fatty acid, wherein
the mineral material is selected from the group comprising natural ground calcium carbonate (GCC) and precipitated calcium carbonate (PCC), and
the hydrophobic antimicrobial agent contains 0.1 to 10 wt% of a copper and/or silver salt of at least one fatty acid, based on the total weight of the mineral material.

2. The hydrophobic antimicrobial agent according to claim 1,
**characterized in that** the natural ground calcium carbonate (GCC) is selected from the group comprising marble, chalk, limestone, and mixtures thereof; and the precipitated calcium carbonate (PCC) is selected from the group comprising precipitated calcium carbonates having aragonitic, vateritic or calcitic crystal forms, and mixtures thereof.

3. The hydrophobic antimicrobial agent according to claim 2,
**characterized in that** the mineral material has
a) a weight median particle size *d*₅₀ in the range from 0.1 µm to 20 µm, preferably from 0.2 µm to 10 µm, more preferably from 0.7 µm to 5 µm and most preferably from 1.5 µm to 3.5 µm, and/or
b) a top cut particle size (*d*₉₈) of not more than 100 µm, preferably not more than 40 µm, even more preferably not more than 25 µm, most preferably not more than 15 µm, and especially of from 2.9 to 13.8 µm, e.g. from 5.8 to 7.4 µm, and/or
c) a specific surface area (BET) of from 0.5 to 50 m²/g, preferably from 1 to 40 m²/g, more preferably from 2 to 30 m²/g, even more preferably from 3 to 20 m²/g, especially from 4 to 15 m²/g and most preferably from 5 to 10 m²/g as measured by the BET nitrogen method.

4. The hydrophobic antimicrobial agent according to any one of the preceding claims,
**characterized in that** the at least one fatty acid is selected from the group comprising aliphatic carboxylic acids having from 5 to 24 carbon atoms, preferably from the group comprising pentanoic acid, hexanoic acid, heptanoic acid, octanoic acid, nonanoic acid, decanoic acid, undecanoic acid, lauric acid, tridecanoic acid, myristic acid, pentadecanoic acid, palmitic acid, heptadecanoic acid, stearic acid, nonadecanoic acid, arachidic acid, heneicosylic acid, behenic acid, tricosylic acid, lignoceric acid and mixtures thereof, more preferably from the group comprising octanoic acid, decanoic acid, lauric acid, myristic acid, palmitic acid, stearic acid, arachidic acid and mixtures thereof and most preferably comprises stearic acid.

5. The hydrophobic antimicrobial agent according to any one of the preceding claims,
**characterized in that** it contains 0.5 to 8 wt%, more preferably 1 to 6 wt%, even more preferably 2 to 5 wt%, most preferably 3 to 4 wt% of a copper and/or silver salt of at least one fatty acid, based on the total weight of the mineral material.

6. The hydrophobic antimicrobial agent according to any one of the preceding claims,
**characterized in that** the hydrophobic antimicrobial agent has a moisture pickup susceptibility of from 0.05 to 1 mg of H₂O/g of solid, preferably from 0.075 to 0.75 mg of H₂O/g of solid, more preferably from 0.1 to 0.5 mg of H₂O/g of solid, even more preferably from 0.15 to 0.40 mg of H₂O/g of solid, and most preferably from 0.2 to 0.3 mg of H₂O/g of solid.

7. Method for the manufacture of the hydrophobic antimicrobial agent according to any one of the preceding claims, **characterized by** the steps of
a) providing at least one fatty acid,
b) providing at least one copper and/or silver salt being soluble in a non-aqueous solvent,
c) providing a mineral material,
d) dissolving the at least one fatty acid of step a) in a non-aqueous solvent,
e) dissolving the at least one copper and/or silver salt of step b) in a non-aqueous solvent,
f) combining the solutions of step d) and e),
g) precipitating the copper and/or silver salt of the at least one fatty acid by adding water to the combined solutions of step f),
h) separating the precipitated copper and/or silver salt of at least one fatty acid from the reaction mixture of step g),
i) surface coating the mineral material of step c) with the precipitated copper and/or silver salt of at least one fatty acid of step h), wherein
the mineral material is selected from the group comprising natural ground calcium carbonate (GCC) and precipitated calcium carbonate (PCC),
the copper and/or silver salt being soluble in a non-aqueous solvent of step b) is selected from the group comprising copper acetate, copper pyrophosphate, copper nitrate, copper chloride, copper oxalate, silver chloride, silver acetate, silver nitrate, and silver sulfate, their hydrates, and mixtures thereof,
the non-aqueous solvent is selected from the group comprising ethanol, methanol, acetone, acetonitrile, dioxane, ethyl acetate, toluene, xylene, tetrahydrofurane, and mixtures thereof,
the combination step f) is carried out under stirring, and
in coating step i), the mineral material of step c) is combined with the copper and/or silver salt of at least one fatty acid of step h) under stirring, wherein the copper and/or silver salt of at least one fatty acid of step h) is combined with the mineral material of step c) in an amount of from 0.1 to 10 wt%, based on the total weight of the mineral material.

8. The method according to claim 7,
**characterized in that**
the at least one copper and/or silver salt of step b) is provided in an amount of from 0.5 to 75 wt%, preferably in an amount of from 1 to 70 wt%, more preferably in an amount of from 5 to 65 wt%, even more preferably in an amount of from 10 to 55 wt%, most preferably in an amount of from 15 to 50 wt%, especially preferably in an amount of from 20 to 40 wt%, e.g. 30 wt% based on the total weight of the at least one fatty acid of step a).

9. The method according to any one of claims 7 to 8,
**characterized in that** the non-aqueous solvent is ethanol.

10. The method according to any one of claims 7 to 9,
**characterized in that** dissolution steps d) and/or e), independently from each other, are carried out at a temperature of from 25 to 80 °C, preferably of from 30 to 70 °C, more preferably of from 40 to 60 °C, most preferably at 50 °C, and/or
combination step f) is carried out under stirring at a temperature of from 25 to 80 °C, preferably of from 30 to 70 °C, more preferably of from 40 to 60 °C, most preferably at 50 °C, and especially preferably at the temperature of any one of steps d) or e).

11. The method according to any one of claims 7 to 10,
**characterized in that,** in coating step i), the mineral material of step c) is combined with the copper and/or silver salt of at least one fatty acid of step h) under stirring at a temperature of from 25 to 180 °C, preferably of from 40 to 160 °C, more preferably of from 60 to 130 °C, most preferably of from 80 to 110 °C, and/or
in coating step i), the copper and/or silver salt of at least one fatty acid of step h) is combined with the mineral material of step c) in an amount of from 0.25 to 8 wt%, preferably in an amount of from 0.5 to 6 wt%, more preferably in an amount of from 0.75 to 4 wt%, most preferably in an amount of from 1 to 2 wt% based on the total weight of the mineral material.

12. Use of the hydrophobic antimicrobial agent according to any one of claims 1 to 6 as a filler, wherein preferably the hydrophobic antimicrobial agent is used as a filler in polymers, paints, adhesives, coatings, rubber, sealants and cosmetics.

13. Product comprising a hydrophobic antimicrobial agent according to any one of claims 1 to 6.

14. Use of a copper and/or silver salt of at least one fatty acid according to any one of claims 1 to 6 for surface coating a mineral material.

## Patentansprüche

1. Hydrophobes antimikrobielles Mittel,
**dadurch gekennzeichnet, dass** es ein mineralisches Material umfasst, das mit einem Kupfer- und/oder Silbersalz von mindestens einer Fettsäure oberflächenbeschichtet ist, wobei
das mineralische Material ausgewählt ist aus der Gruppe umfassend natürliches gemahlenes Calciumcarbonat (GCC) und gefälltes Calciumcarbonat (PCC), und
das hydrophobe antimikrobielle Mittel 0,1 bis 10 Gew.-% eines Kupfer- und/oder Silbersalzes von mindestens einer Fettsäure enthält, bezogen auf das Gesamtgewicht des mineralischen Materials.

2. Hydrophobes antimikrobielles Mittel nach Anspruch 1,
**dadurch gekennzeichnet, daß** das natürliche gemahlene Calciumcarbonat (GCC) aus der Gruppe ausgewählt ist umfassend Marmor, Kreide, Kalkstein und Mischungen davon; und das gefällte Calciumcarbonat (PCC) aus der Gruppe ausgewählt ist umfassend gefälltes Calciumcarbonat mit aragonitischen, vateritischen oder calcitischen Kristallformen und Mischungen davon.

3. Hydrophobes antimikrobielles Mittel nach Anspruch 2,
**dadurch gekennzeichnet, dass** das mineralische Material aufweist
a) eine Gewichts-Median-Partikelgröße *d*₅₀ im Bereich von 0,1 µm bis 20 µm, vorzugsweise von 0,2 µm bis 10 µm, besonders bevorzugt von 0,7 µm bis 5 µm und am meisten bevorzugt von 1,5 µm bis 3,5 µm, und/oder
b) eine Top-Cut-Partikelgröße (*d*₉₈) von nicht mehr als 100 µm, vorzugsweise nicht mehr als 40 µm, besonders bevorzugt nicht mehr als 25 µm, am meisten bevorzugt nicht mehr als 15 µm, und insbesondere von 2,9 bis 13,8 µm, z.B. von 5,8 bis 7,4 µm, und/oder
c) eine spezifische Oberfläche (BET) von 0,5 bis 50 m²/g, vorzugsweise von 1 bis 40 m²/g, besonders bevorzugt von 2 bis 30 m²/g, noch stärker bevorzugt von 3 bis 20 m²/g, insbesondere von 4 bis 15 m²/g und am meisten bevorzugt von 5 bis 10 m²/g, gemessen nach der BET-Stickstoffmethode.

4. Hydrophobes antimikrobielles Mittel nach einem der vorhergehenden Ansprüche,
**dadurch gekennzeichnet, dass** die mindestens eine Fettsäure aus der Gruppe ausgewählt ist umfassend aliphatische Carbonsäuren mit 5 bis 24 Kohlenstoffatomen, vorzugsweise aus der Gruppe umfassend Pentansäure, Hexansäure, Heptansäure, Octansäure, Nonansäure, Decansäure, Undecansäure, Laurinsäure, Tridecansäure, Myristinsäure, Pentadecansäure, Palmitinsäure, Pentadecansäure, Palmitinsäure, Heptadecansäure, Stearinsäure, Nonadecansäure, Arachidinsäure, Henicosylsäure, Behensäure, Tricosylsäure, Lignocerinsäure und Mischungen davon, besonders bevorzugt aus der Gruppe umfassend Octansäure, Decansäure, Laurinsäure, Myristinsäure, Palmitinsäure, Stearinsäure, Arachidinsäure und Mischungen davon, und am meisten bevorzugt umfassend Stearinsäure.

5. Hydrophobes antimikrobielles Mittel nach einem der vorhergehenden Ansprüche,
**dadurch gekennzeichnet, dass** es 0,5 bis 8 Gew.-%, vorzugsweise 1 bis 6 Gew.-%, besonders bevorzugt 2 bis 5 Gew.-%, am meisten bevorzugt 3 bis 4 Gew.-% eines Kupfer- und/oder Silbersalzes von mindestens einer Fettsäure enthält, bezogen auf das Gesamtgewicht des mineralischen Materials.

6. Hydrophobes antimikrobielles Mittel nach einem der vorhergehenden Ansprüche,
**dadurch gekennzeichnet, daß** das hydrophobe antimikrobielle Mittel eine Feuchtigkeitsaufnahme-Suszeptibilität von 0,05 bis 1 mg H₂O/g Feststoff aufweist, vorzugsweise von 0,075 bis 0,75 mg H₂O/g Feststoff, besonders bevorzugt von 0,1 bis 0,5 mg H₂O/g Feststoff, noch stärker bevorzugt von 0,15 bis 0,40 mg H₂O/g Feststoff und am meisten bevorzugt von 0,2 bis 0,3 mg H₂O/g Feststoff.

7. Verfahren zur Herstellung des hydrophoben antimikrobiellen Mittels nach einem der vorhergehenden Ansprüche, **gekennzeichnet durch** die Schritte
a) Bereitstellen mindestens einer Fettsäure,
b) Bereitstellen mindestens eines Kupfer- und/oder Silbersalzes, das in einem nichtwässrigen Lösungsmittel löslich ist,
c) Bereitstellen eines mineralischen Materials,
d) Lösen der mindestens einen Fettsäure aus Schritt a) in einem nichtwässrigen Lösungsmittel,
e) Lösen des mindestens einen Kupfer- und/oder Silbersalzes aus Schritt b) in einem nichtwässrigen Lösungsmittel,
f) Vereinigen der Lösungen von Schritt d) und e),
g) Fällen des Kupfer- und/oder Silbersalzes der mindestens einen Fettsäure durch Zugabe von Wasser zu den vereinigten Lösungen von Schritt f),
h) Abtrennen des ausgefällten Kupfer- und/oder Silbersalzes der mindestens einen Fettsäure aus dem Reaktionsgemisch von Schritt g),
i) Oberflächenbeschichten des mineralischen Materials von Schritt c) mit dem ausgefällten Kupfer- und/oder Silbersalz der mindestens einen Fettsäure von Schritt h), wobei
das mineralische Material ausgewählt ist aus der Gruppe umfassend natürliches gemahlenes Calciumcarbonat (GCC) und gefälltes Calciumcarbonat (PCC),
das Kupfer- und/oder Silbersalz von Schritt b), das in einem nichtwässrigen Lösungsmittel löslich ist, aus der Gruppe ausgewählt ist umfassend Kupferacetat, Kupferpyrophosphat, Kupfernitrat, Kupferchlorid, Kupferoxalat, Silberchlorid, Silberacetat, Silbernitrat und Silbersulfat, deren Hydrate und Mischungen davon,
das nichtwässrige Lösungsmittel ausgewählt ist aus der Gruppe umfassend Ethanol, Methanol, Aceton, Acetonitril, Dioxan, Ethylacetat, Toluol, Xylol, Tetrahydrofuran und Mischungen davon,
der Vereinigungsschritt f) unter Rühren durchgeführt wird, und
im Beschichtungsschritt i) das mineralische Material von Schritt c) mit dem Kupfer- und/oder Silbersalz der mindestens einen Fettsäure von Schritt h) unter Rühren vereinigt wird, wobei das Kupfer- und/oder Silbersalz der mindestens einen Fettsäure von Schritt h) mit dem mineralischen Material von Schritt c) in einer Menge von 0,1 bis 10 Gew.-%, bezogen auf das Gesamtgewicht des mineralischen Materials, vereinigt wird.

8. Verfahren nach Anspruch 7,
**dadurch gekennzeichnet, dass**
das mindestens eine Kupfer- und/oder Silbersalz aus Schritt b) in einer Menge von 0,5 bis 75 Gew.-%, vorzugsweise in einer Menge von 1 bis 70 Gew.-%, besonders bevorzugt in einer Menge von 5 bis 65 Gew.-%, noch stärker bevorzugt in einer Menge von 10 bis 55 Gew.-%, am meisten bevorzugt in einer Menge von 15 bis 50 Gew.-%, insbesondere bevorzugt in einer Menge von 20 bis 40 Gew.-%, z.B. 30 Gew.-%, bezogen auf das Gesamtgewicht der mindestens einen Fettsäure aus Schritt a), bereitgestellt wird.

9. Verfahren nach einem der Ansprüche 7 bis 8,
**dadurch gekennzeichnet, dass** das nichtwässrige Lösungsmittel Ethanol ist.

10. Verfahren nach einem der Ansprüche 7 bis 9,
**dadurch gekennzeichnet, dass** die Lösungsschritte d) und/oder e) unabhängig voneinander bei einer Temperatur von 25 bis 80 °C, vorzugsweise von 30 bis 70 °C, besonders bevorzugt von 40 bis 60 °C, am meisten bevorzugt bei 50 °C durchgeführt werden und/oder
der Vereinigungsschritt f) unter Rühren bei einer Temperatur von 25 bis 80 °C, vorzugsweise von 30 bis 70 °C, besonders bevorzugt von 40 bis 60 °C, am meisten bevorzugt bei 50 °C, und insbesondere bevorzugt bei der Temperatur eines der Schritte d) oder e) durchgeführt wird.

11. Verfahren nach einem der Ansprüche 7 bis 10,
**dadurch gekennzeichnet, dass** in dem Beschichtungsschritt i) das mineralische Material der Stufe c) mit dem Kupfer- und/oder Silbersalz der mindestens einen Fettsäure der Stufe h) unter Rühren bei einer Temperatur von 25 bis 180 °C, vorzugsweise von 40 bis 160 °C, besonders bevorzugt von 60 bis 130 °C, am meisten bevorzugt von 80 bis 110 °C vereinigt wird und/oder
in Beschichtungsschritt i) das Kupfer- und/oder Silbersalz der mindestens einen Fettsäure aus Schritt h) mit dem mineralische Material aus Schritt c) in einer Menge von 0,25 bis 8 Gew.-%, vorzugsweise in einer Menge von 0,5 bis 6 Gew.-%, besonders bevorzugt in einer Menge von 0,75 bis 4 Gew.-%, am meisten bevorzugt in einer Menge von 1 bis 2 Gew.-%, bezogen auf das Gesamtgewicht des mineralischen Materials, vereinigt wird.

12. Verwendung des hydrophoben antimikrobiellen Mittels nach einem der Ansprüche 1 bis 6 als Füllstoff, wobei das hydrophobe antimikrobielle Mittel vorzugsweise als Füllstoff in Polymeren, Farben, Klebstoffen, Beschichtungen, Gummi, Dichtungsmitteln und Kosmetika verwendet wird.

13. Produkt umfassend ein hydrophobes antimikrobielles Mittel nach einem der Ansprüche 1 bis 6.

14. Verwendung eines Kupfer- und/oder Silbersalzes vom mindestens einer Fettsäure nach einem der Ansprüche 1 bis 6 zur Oberflächenbeschichtung eines mineralischen Materials.

## Revendications

1. Agent antimicrobien hydrophobe,
**caractérisé en ce qu'**il comprend une matière minérale, qui est recouverte en surface d'un sel de cuivre et/ou d'argent d'au moins un acide gras, dans lequel la matière minérale est choisie dans le groupe comprenant le carbonate de calcium naturel broyé (GCC) et le carbonate de calcium précipité (PCC), et l'agent antimicrobien hydrophobe contient de 0,1 à 10 % en poids d'un sel de cuivre et/ou d'argent d'au moins un acide gras, en se basant sur le poids total de la matière minérale.

2. Agent antimicrobien hydrophobe selon la revendication 1,
**caractérisé en ce que** le carbonate de calcium naturel broyé (GCC) est choisi dans le groupe comprenant le marbre, la craie, le calcaire et des mélanges de ceux-ci ; et le carbonate de calcium précipité (PCC) est choisi dans le groupe comprenant les carbonates de calcium précipités ayant des formes cristallines aragonitiques, vatéritiques ou calcitiques, et des mélanges de celles-ci.

3. Agent antimicrobien hydrophobe selon la revendication 2,
**caractérisé en ce que** la matière minérale a
a) une granulométrie moyenne en poids *d*₅₀ dans la plage allant de 0,1 µm à 20 µm, de préférence de 0,2 µm à 10 µm, plus préférablement de 0,7 µm à 5 µm et de manière préférée entre toutes de 1,5 µm à 3,5 µm, et/ou
b) une granulométrie de coupe supérieure (*d*₉₈) de pas plus de 100 µm, de préférence de pas plus de 40 µm, encore plus préférablement de pas plus de 25 µm, de manière préférée entre toutes de pas plus de 15 µm, et en particulier de 2,9 à 13,8 µm, par ex. de 5,8 à 7,4 µm, et/ou
c) une surface spécifique (BET) de 0,5 à 50 m²/g, de préférence de 1 à 40 m²/g, plus préférablement de 2 à 30 m²/g, encore plus préférablement de 3 à 20 m²/g, en particulier de 4 à 15 m²/g et de manière préférée entre toutes de 5 à 10 m²/g comme mesuré par la méthode BET à base d'azote.

4. Agent antimicrobien hydrophobe selon l'une quelconque des revendications précédentes,
**caractérisé en ce que** l'au moins un acide gras est choisi dans le groupe comprenant les acides carboxyliques aliphatiques ayant de 5 à 24 atomes de carbone, de préférence dans le groupe comprenant l'acide pentanoïque, l'acide hexanoïque, l'acide heptanoïque, l'acide octanoïque, l'acide nonanoïque, l'acide décanoïque, l'acide undécanoïque, l'acide laurique, l'acide tridécanoïque, l'acide myristique, l'acide pentadécanoïque, l'acide palmitique, l'acide heptadécanoïque, l'acide stéarique, l'acide nonadécanoïque, l'acide arachidique, l'acide hénéicosylique, l'acide béhénique, l'acide tricosylique, l'acide lignocérique et des mélanges de ceux-ci, plus préférablement dans le groupe comprenant l'acide octanoïque, l'acide décanoïque, l'acide laurique, l'acide myristique, l'acide palmitique, l'acide stéarique, l'acide arachidique et des mélanges de ceux-ci et de manière préférée entre toutes comprend l'acide stéarique.

5. Agent antimicrobien hydrophobe selon l'une quelconque des revendications précédentes,
**caractérisé en ce qu'**il contient 0,5 à 8 % en poids, plus préférablement 1 à 6 % en poids, encore plus préférablement 2 à 5 % en poids, de manière préférée entre toutes 3 à 4 % en poids d'un sel de cuivre et/ou d'argent d'au moins un acide gras, en se basant sur le poids total de la matière minérale.

6. Agent antimicrobien hydrophobe selon l'une quelconque des revendications précédentes,
**caractérisé en ce que** l'agent antimicrobien hydrophobe a une sensibilité à l'humidité de 0,05 et 1 mg d'H₂O/g de solide, de préférence de 0,075 à 0,75 mg d'H₂O/g de solide, plus préférablement de 0,1 à 0,5 mg d'H₂O/g de solide, encore plus préférablement de 0,15 à 0,40 mg d'H₂O/g de solide, et de manière préférée entre toutes de 0,2 à 0,3 mg d'H₂O/g de solide.

7. Procédé de fabrication de l'agent antimicrobien hydrophobe selon l'une quelconque des revendications précédentes, **caractérisé par** les étapes consistant à
a) fournir au moins un acide gras,
b) fournir au moins un sel de cuivre et/ou d'argent soluble dans un solvant non aqueux,
c) fournir une matière minérale,
d) dissoudre l'au moins un acide gras de l'étape a) dans un solvant non aqueux,
e) dissoudre l'au moins un sel de cuivre et/ou d'argent de l'étape b) dans un solvant non aqueux,
f) combiner les solutions des étapes d) et e),
g) précipiter le sel de cuivre et/ou d'argent de l'au moins un acide gras par ajout d'eau aux solutions combinées de l'étape f),
h) séparer le sel de cuivre et/ou d'argent précipité de l'au moins un acide gras du mélange réactionnel de l'étape g),
i) revêtir en surface la matière minérale de l'étape c) avec le sel de cuivre et/ou d'argent précipité de l'au moins un acide gras de l'étape h), dans lequel
la matière minérale est choisie dans le groupe comprenant le carbonate de calcium naturel broyé (GCC) et le carbonate de calcium précipité (PCC),
le sel de cuivre et/ou d'argent soluble dans un solvant non aqueux de l'étape b) est choisi dans le groupe comprenant l'acétate de cuivre, le pyrophosphate de cuivre, le nitrate de cuivre, le chlorure de cuivre, l'oxalate de cuivre, le chlorure d'argent, l'acétate d'argent, le nitrate d'argent et le sulfate d'argent, leurs hydrates et des mélanges de ceux-ci,
le solvant non aqueux est choisi dans le groupe comprenant l'éthanol, le méthanol, l'acétone, l'acétonitrile, le dioxane, l'acétate d'éthyle, le toluène, le xylène, le tétrahydrofurane et des mélanges de ceux-ci,
l'étape de combinaison f) est réalisée sous agitation, et
lors de l'étape de revêtement i), la matière minérale de l'étape c) est combinée avec le sel de cuivre et/ou d'argent d'au moins un acide gras de l'étape h) sous agitation, dans lequel le sel de cuivre et/ou d'argent d'au moins un acide gras de l'étape h) est combiné avec la matière minérale de l'étape c) en une quantité de 0,1 à 10 % en poids, en se basant sur le poids total de la matière minérale.

8. Procédé selon la revendication 7,
**caractérisé en ce que**
l'au moins un sel de cuivre et/ou d'argent de l'étape b) est fourni en une quantité de 0,5 à 75 % en poids, de préférence en une quantité de 1 à 70 % en poids, plus préférablement en une quantité de 5 à 65 % en poids, encore plus préférablement en une quantité de 10 à 55 % en poids, de manière préférée entre toutes en une quantité de 15 à 50 % en poids, en particulier de préférence en une quantité de 20 à 40 % en poids, par ex. 30 % en poids en se basant sur le poids total de l'au moins un acide gras de l'étape a).

9. Procédé selon l'une quelconque des revendications 7 à 8,
**caractérisé en ce que** le solvant non aqueux est de l'éthanol.

10. Procédé selon l'une quelconque des revendications 7 à 9,
**caractérisé en ce que** les étapes de dissolution d) et/ou e), indépendamment l'une de l'autre, sont réalisées à une température de 25 à 80 °C, de préférence de 30 à 70 °C, plus préférablement de 40 à 60 °C, de manière préférée entre toutes à 50 °C, et/ou
l'étape de combinaison f) est réalisée sous agitation à une température de 25 à 80 °C, de préférence de 30 à 70 °C, plus préférablement de 40 à 60 °C, de manière préférée entre toutes à 50 °C, et en particulier de préférence à la température de l'une quelconque des étapes d) ou e).

11. Procédé selon l'une quelconque des revendications 7 à 10,
**caractérisé en ce que**, lors de l'étape de revêtement i), la matière minérale de l'étape c) est combinée avec le sel de cuivre et/ou d'argent d'au moins un acide gras de l'étape h) sous agitation à une température de 25 à 180 °C, de préférence de 40 à 160 °C, plus préférablement de 60 à 130 °C, de manière préférée entre toutes de 80 à 110 °C, et/ou
lors de l'étape de revêtement i), le sel de cuivre et/ou d'argent d'au moins un acide gras de l'étape h) est combiné avec la matière minérale de l'étape c) en une quantité de 0,25 à 8 % en poids, de préférence en une quantité de 0,5 à 6 % en poids, plus préférablement en une quantité de 0,75 à 4 % en poids, de manière préférée entre toutes en une quantité de 1 à 2 % en poids en se basant sur le poids total de la matière minérale.

12. Utilisation de l'agent antimicrobien hydrophobe selon l'une quelconque des revendications 1 à 6 comme charge, dans laquelle de préférence l'agent antimicrobien hydrophobe est utilisé comme charge dans les polymères, les peintures, les adhésifs, les revêtements, le caoutchouc, les produits d'étanchéité et les cosmétiques.

13. Produit comprenant un agent antimicrobien hydrophobe selon l'une quelconque des revendications 1 à 6.

14. Utilisation d'un sel de cuivre et/ou d'argent d'au moins un acide gras selon l'une quelconque des revendications 1 à 6 pour le revêtement de surface d'une matière minérale.
